# FASCICULE DE BREVET EUROPEEN

(11) **EP 2 486 022 B1**
(45) Date de publication et mention de la délivrance du brevet: **05.08.2015**
(21) Numéro de dépôt: 10762923.0
(22) Date de dépôt: 08.10.2010
(51) Int. Cl.: C07D 253/07, C08K 5/00

(54) **1, 2, 4 - TRIAZINE UTILISABLE COMME ACCÉLÉRATEUR DE VULCANISATION ET SON PROCÉDÉ D'OBTENTION**
1,2,4-TRIAZIN ALS VULKANISIERUNGSBESCHLEUNIGER UND VERFAHREN ZU DESSEN HERSTELLUNG
1, 2, 4 - TRIAZINE SUITABLE AS A VULCANIZATION ACCELERATOR AND METHOD FOR PRODUCING SAME

(30) Priorité: 08.10.2009 FR 0957036
(43) Date de publication de la demande: 15.08.2012
(73) Titulaire: COMPAGNIE GENERALE DES ETABLISSEMENTS MICHELIN, 63000 Clermont-Ferrand (FR); MICHELIN Recherche et Technique S.A., 1763 Granges-Paccot (CH)
(72) Inventeur: SEEBOTH, Nicolas, F-63000 Clermont-Ferrand (FR); IVANOV, Sergey, Orekhovo-Zouevo 142611 (RU); MOLKOV, Sergey, Tcheboksary 428031 (RU)
(74) Mandataire: Sidhu, Alban
(86) Numéro de dépôt international: PCT/EP2010/065067
(87) Numéro de publication internationale: WO 2011/042521

(56) Documents cités:
- US-A- 3 211 729
- US-A- 4 301 260

## Description

La présente invention se rapporte à une triazine particulière, à son procédé d'obtention et à son utilisation en tant qu'accélérateur de vulcanisation.

La vulcanisation des élastomères diéniques par le soufre est largement utilisée dans l'industrie du caoutchouc, en particulier celle du pneumatique. Pour vulcaniser les élastomères diéniques, on utilise un système de vulcanisation relativement complexe comportant, en plus du soufre, un accélérateur primaire de vulcanisation, tels que les sulfénamides à noyau benzothiazole, ainsi que divers accélérateurs secondaires ou activateurs de vulcanisation, tout particulièrement des dérivés du zinc tels que l'oxyde de zinc (ZnO) seul ou utilisé avec des acides gras.

Le document US 3 211 729 décrit des composés 1,2,4-triazine substitués en position 3, 5 et 6 de la triazine par des radicaux benzène ou pyridine. Ces composés peuvent être utilisés comme agent de protection solaire dans des matériaux organiques.

Le document US 4 301 260 décrit des compositions de caoutchouc vulcanisables comprenant des composés 1,3,5-triazine.

De tels accélérateurs de vulcanisation, pour être utilisés dans des compositions de caoutchouc à base d'élastomères diéniques et de charges renforçantes utilisables notamment pour la fabrication de pneumatiques, doivent induire une réticulation suffisante tout en conservant une phase retard (temps nécessaire au début de la vulcanisation) correcte voire améliorée.

Les accélérateurs de vulcanisation jouent un rôle important dans l'obtention d'une phase retard (délai d'induction), et il est connu de l'homme du métier que ce paramètre est très difficile à ajuster. Il est donc particulièrement intéressant pour l'homme du métier d'avoir un accélérateur de vulcanisation induisant une phase retard longue, qu'il pourra ajuster s'il le souhaite par l'ajout d'accélérateurs complémentaires.

La demanderesse a découvert qu'un composé triazine particulier, la 1,2,4-triazine, pouvait être utilisé comme accélérateur de vulcanisation et permettait d'améliorer encore les propriétés rhéométriques d'une composition de caoutchouc utilisant un tel composé, sans détériorer les autres propriétés d'une telle composition.

L'invention a donc pour objet une 1,2,4-triazine de formule (I) : 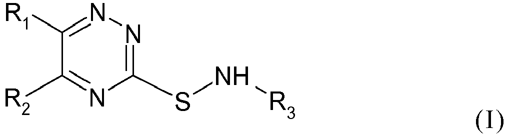 où
R₁ et R₂ représentent indépendamment H ou un groupe hydrocarboné en C₁-C₂₅ choisi parmi les groupes alkyles linéaires, ramifiés ou cycliques, et les groupes aryles, R₁ et R₂ pouvant former ensemble un cycle,
R₃ représente :
   - un groupe alkyle en C₁-C₂₅, linéaire ou ramifié, éventuellement substitué par un ou plusieurs groupes alkyles cycliques en C₃-C₁₀ ou aryles en C₆-C₁₂, ou
   - un groupe alkyle cyclique en C₃-C₁₀, éventuellement substitué par un ou plusieurs groupes alkyles linéaires, ramifiés ou cycliques en C₁-C₂₅ ou aryles en C₆-C₁₂.

L'invention a également pour objet l'utilisation, à titre d'accélérateur de vulcanisation, d'une 1,2,4-triazine telle que définie précédemment.

L'invention a encore pour objet un procédé de préparation d'une 1,2,4-triazine telle que définie précédemment par couplage oxydant entre
- un composé 1,2,4-triazine-3-thiol de formule (A) suivante : 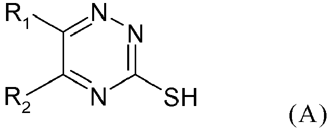 où R₁ et R₂ sont tels que définis précédemment, et
- une amine primaire de formule R₃NH₂, où R₃ est tel que défini précédemment,
le couplage oxydant étant réalisé au moyen d'une composition basique et d'un composé oxydant.

L'invention ainsi que ses avantages seront aisément compris à la lumière de la description et des exemples de réalisation qui suivent.

### I. Mesures et tests utilisés

Les compositions de caoutchouc, dans lesquelles sont testés les accélérateurs de vulcanisation 1,2,4-triazines, sont caractérisées, après cuisson, comme indiqué ci-après.

### Rhéométrie

Les mesures sont effectuées à 150°C avec un rhéomètre à chambre oscillante, selon la norme DIN 53529 - partie 3 (juin 1983). L'évolution du couple rhéométrique, ΔCouple, en fonction du temps décrit l'évolution de la rigidification de la composition par suite de la réaction de vulcanisation. Les mesures sont traitées selon la norme DIN 53529 - partie 2 (mars 1983) : T₀ est le délai d'induction, c'est-à-dire le temps nécessaire au début de la réaction de vulcanisation ; T_{α} (par exemple T₉₉) est le temps nécessaire pour atteindre une conversion de α%, c'est-à-dire α% (par exemple 99%) de l'écart entre les couples minimum et maximum. On mesure également la constante de vitesse de conversion notée K (exprimée en min-1), d'ordre 1, calculée entre 30% et 80% de conversion, qui permet d'apprécier la cinétique de vulcanisation.

### II. Conditions de réalisation de l'invention

### II-1. Triazine de l'invention

Comme expliqué précédemment, le premier objet de l'invention est une 1,2,4-triazine de formule (I) suivante : 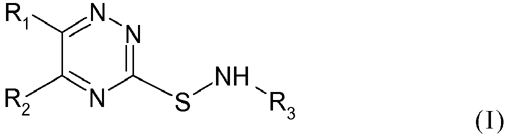 où
R₁ et R₂ représentent indépendamment H ou un groupe hydrocarboné en C₁-C₂₅ choisi parmi les groupes alkyles linéaires, ramifiés ou cycliques, et les groupes aryles, R₁ et R₂ Pouvant former ensemble un cycle,
R₃ représente :
   - un groupe alkyle en C₁-C₂₅, linéaire ou ramifié, éventuellement substitué par un ou plusieurs groupes alkyles cycliques en C₃-C₁₀ ou aryles en C₆-C₁₂, ou
   - un groupe alkyle cyclique en C₃-C₁₀, éventuellement substitué par un ou plusieurs groupes alkyles linéaires, ramifiés ou cycliques en C₁-C₂₅ ou aryles en C₆-C₁₂.

Par groupe alkyle cyclique, on entend un groupe alkyle constitué d'un ou plusieurs cycles.

Selon un premier mode de réalisation, R₁ et R₂ représentent indépendamment H, un groupe méthyle ou un groupe phényle.

Avantageusement, R₁ ou R₂ représentent un groupe phényle. De préférence, R₂ est un groupe phényle et R₁ est un hydrogène.

R₃ peut représenter un groupe cyclohexyle ou un groupe tertbutyle. De préférence, R₃ représente un groupe tertbutyle.

Une 1,2,4-triazine préférée de formule (I) est celle dans laquelle R₁ représente l'hydrogène, R₂ représente un groupe phényle et R₃ représente un groupe cyclohexyle. Un tel composé est la 3-[(cyclohexylamino)thio]-5-phényl-1,2,4-triazine (encore appelée N-cyclohexyl-S-(5-phényl-1,2,4-triazin-3-yl)thiohydroxylamine).

Une autre 1,2,4-triazine préférée de formule (I) est celle dans laquelle R₁ représente l'hydrogène, R₂ représente un groupe phényle et R₃ représente un groupe tertbutyle. Un tel composé est la 3-[(t-butylamino)thio]-5-phényl-1,2,4-triazine.

### II-2. Procédé de synthèse

Les 1,2,4-triazines de formule (I) peuvent être préparés par couplage oxydant entre
- un composé 1,2,4-triazine-3-thiol de formule (A) suivante : 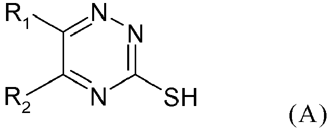 où R₁ et R₂ sont tels que définis précédemment, et
- une amine primaire de formule R₃NH₂, où R₃ est tel que défini précédemment,
le couplage oxydant étant réalisé au moyen d'une composition basique et d'un composé oxydant.

Selon un premier mode de réalisation de l'invention, les 1,2,4-triazines de formule (I) peuvent être préparés selon un procédé de synthèse comportant les étapes suivantes :
- on part du composé 1,2,4-triazine-3-thiol de formule (A) suivante : 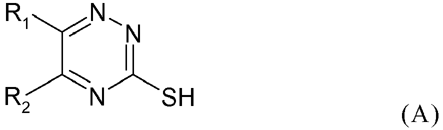
   où R₁ et R₂ sont tels que définis précédemment,
   ce 1,2,4-triazine-3-thiol pouvant être obtenu, sans que ce cela soit limitatif, par condensation entre un composé 1,2 dicarbonylé et le thiosemicarbazide selon des méthodes génériques décrites par exemple dans le Journal of Organic Chemistry, Vol 52, n° 19, 1987, p4280-4287 ou encore par L. M. Mironovich et V.K. Promonenkov dans le chapitre intitulé 1,2,4-Triazines du volume 22 de l'ouvrage Bilans de la science et la technique. Série Chimie organique édité en 1990 par Viniti à Moscou ;
- on fait réagir le composé (A) avec la composition basique qui peut être une solution aqueuse d'une base organique ou minérale, par exemple une solution aqueuse d'hydroxyde de sodium, puis
- on ajoute au milieu réactionnel l'amine primaire de formule R₃NH₂ ou R₃ est tel que défini précédemment, et on réalise le couplage oxydant en présence du composé oxydant qui peut être par exemple un composé halogéné tel que le dichlore, le dibrome, le diiode, les acides hypohalogéneux ou encore leurs sels alcalins tels que par exemple l'hypochlorite de sodium.

De préférence, R₁ représente l'hydrogène et R₂ représente un groupe phényle.

De préférence, R₃ représente un groupe cyclohexyle.

Selon un deuxième mode de réalisation de l'invention, les 1,2,4-triazines de formule (I) peuvent être préparés selon un procédé de synthèse comportant les étapes suivantes :
a) on fait réagir de l'amine primaire de formule R₃NH₂ avec ledit composé oxydant, puis
b) on ajoute au milieu réactionnel obtenu à l'étape a) une composition comprenant la composition basique, ledit composé de formule (A) et de l'amine primaire de formule R₃NH₂.

De préférence, R₁ représente l'hydrogène et R₂ représente un groupe phényle.

De préférence, R₃ représente un groupe tertbutyle.

La composition basique peut être une solution aqueuse d'une base organique ou minérale, par exemple une solution aqueuse d'hydroxyde de sodium.

Le composé oxydant peut être choisi parmi les composés halogénés, de préférence le dichlore, le dibrome, le diiode et les acides hypohalogéneux et leurs sels alcalins. On peut citer en particulier l'hypochlorite de sodium.

### II-3. Utilisation à titre d'accélérateur de vulcanisation

Comme indiqué précédemment, le composé 1,2,4-triazine de l'invention trouve une application industrielle avantageuse comme accélérateur de vulcanisation. Il peut donc être utilisé dans une composition de caoutchouc pour la fabrication de pneumatiques, à base d'un ou plusieurs élastomères diéniques, d'une ou plusieurs charges renforçantes et d'un système de vulcanisation.

Pour une telle utilisation, le ou les élastomères diéniques sont choisis préférentiellement dans le groupe des élastomères diéniques fortement insaturés constitué par les polybutadiènes (en abrégé "BR"), les polyisoprènes (IR) de synthèse, le caoutchouc naturel (NR), les copolymères de butadiène, les copolymères d'isoprène et les mélanges de ces élastomères. De tels copolymères sont plus préférentiellement choisis dans le groupe constitué par les copolymères de butadiène-styrène (SBR), les copolymères d'isoprène-butadiène (BIR), les copolymères d'isoprène-styrène (SIR) et les copolymères d'isoprène-butadiène-styrène (SBIR).

Par ailleurs, on peut utiliser tout type de charge renforçante connue pour ses capacités à renforcer une composition de caoutchouc utilisable pour la fabrication de pneumatiques, par exemple une charge organique tel que du noir de carbone, une charge inorganique renforçante telle que de la silice, ou encore un coupage de ces deux types de charge, notamment un coupage de noir de carbone et de silice.

Par "charge inorganique renforçante", doit être entendu dans la présente demande, par définition, toute charge inorganique ou minérale (quelles que soient sa couleur et son origine (naturelle ou de synthèse), encore appelée charge "blanche", charge "claire" voire "charge non noire" ("non-black filler") par opposition au noir de carbone, capable de renforcer à elle seule, sans autre moyen qu'un agent de couplage intermédiaire, une composition de caoutchouc destinée à la fabrication de pneumatiques, en d'autres termes apte à remplacer, dans sa fonction de renforcement, un noir de carbone conventionnel de grade pneumatique ; une telle charge se caractérise généralement, de manière connue, par la présence de groupes hydroxyle (-OH) à sa surface.

Comme charges inorganiques renforçantes conviennent notamment des charges minérales du type siliceuse, en particulier de la silice (SiO₂).

Le système de vulcanisation proprement dit est à base de soufre (ou d'un agent donneur de soufre) et d'un accélérateur primaire de vulcanisation. A ce système de vulcanisation de base viennent s'ajouter divers accélérateurs secondaires ou activateurs de vulcanisation connus tels qu'oxyde de zinc, acide stéarique ou composés équivalents, dérivés guanidiques (en particulier diphénylguanidine).

L'accélérateur primaire de vulcanisation doit permettre une réticulation des compositions de caoutchouc dans des temps industriellement acceptables, tout en préservant un délai minimum de sécurité (« temps de grillage ») au cours duquel les compositions peuvent être mises en forme sans risque de vulcanisation prématurée (« grillage »).

Le composé 1,2,4-triazine selon l'invention peut donc être utilisé à titre d'accélérateur de vulcanisation. Il remplace en tout ou partie les composés habituels, notamment les sulfénamides.

### III. Exemples de réalisation de l'invention

### III-1 La 3-[(cyclohexylamino)thio]-5-phényl-1,2.4-triazine

Dans les exemples qui suivent, l'invention est mise en oeuvre avec la 3-[(cyclohexylamino)thio]-5-phényl-1,2,4-triazine (composé B) de formule suivante :

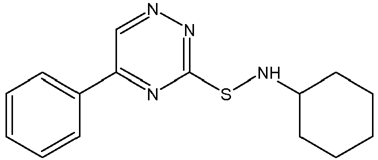

### III-1.1 Synthèse du composé triazine

La préparation de ce composé est effectuée à partir du 5-phényl-1,2,4-triazine-3-thiol et de la cyclohexylamine, selon le schéma de synthèse suivant :

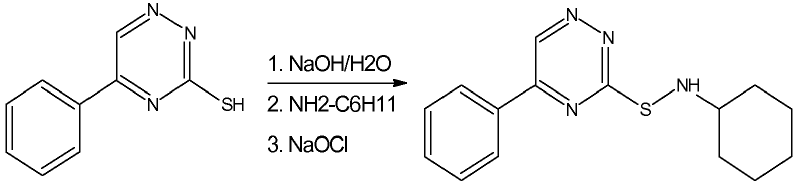

Le 5-phényl-1,2,4-triazine-3-thiol (numéro CAS [15969-28-5] est commercial et peut être obtenu selon des procédures décrites dans les documents suivants :
1. Daunis, J. et al.; Bulletin de la Société Chimique de France; 1969, 10; 3675 - 3678.
2. Joshi, K. C.; Dubey, K. Dandia, A. Heterocycles, (1981), 16(9); 1545 - 1553.

A une solution de 5-phényl-1,2,4-triazine-3-thiol (41,0 g, 0,22 mol) et d'hydroxyde de sodium (20,0 g, 0,50 mol) dans l'eau (700 mL) est ajouté la cyclohexylamine (107,6 g, 1,09 mol). Le mélange est refroidi jusqu'à une température comprise entre 0 et -5°C puis est ajoutée goutte à goutte la solution aqueuse de NaOCl (4 % de chlore actif) (477 mL) pendant 30 minutes. La température du milieu réactionnel reste comprise entre 0 et - 4°C. Le milieu réactionnel est ensuite agité pendant 1h30 à 2 heures à une température comprise entre 0 et 5°C.

De l'éther de pétrole (100 mL) est ajouté et le milieu réactionnel est ensuite agité pendant 15 à 30 minutes à une température comprise entre 0 et -4°C. Le précipité est filtré et lavé par de l'eau (200 mL) et de l'éther de pétrole (50 mL) et enfin séché pendant 2 à 3 heures sous pression réduite.

Un solide blanc (42,9 g, 0,15 mol, rendement 68 %) de point de fusion 125-127 °C est obtenu.

La pureté molaire est supérieure à 97 % (RMN ¹H).

Si la pureté n'est pas suffisante, une cristallisation dans l'acétate d'éthyle permet d'obtenir le produit pur.

La caractérisation complète du produit est réalisée par RMN. Les déplacements chimiques obtenus par RMN ¹H et ¹³C dans le DMSO-d6 sont donnés dans le tableau ci-dessous. La calibration est réalisée sur le DMSO (2,44 ppm en ¹H et à 39,5 ppm en ¹³C).

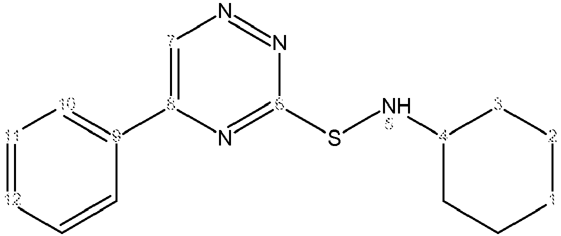

Les résultats sont indiqués dans le tableau 1.

**Tableau 1**

| *N°* | 1 | 2 | 3 | 4 | 5 | 6 | 7 | 8 | 9 | 10 | 11 | 12 |
|---|---|---|---|---|---|---|---|---|---|---|---|---|
| *δ(¹H) en ppm* | 1,45 1,13 | 1,63 1,13 | 1,92 1,13 | 2,97 | 5,08 | / | 9,73 | / | / | 8,27 | 7,56 | 7,61 |
| *δ(¹³C) en ppm* | 25,5 | 23,8 | 32,4 | 56,7 | / | 176,4 | 143,2 | 154,2 | 132,9 | 127,8 | 129,3 | 132,8 |

### III-1.2. Utilisation comme accélérateur de vulcanisation-Préparation des compositions

On procède pour les essais qui suivent de la manière suivante: on introduit dans un mélangeur interne, rempli à 70% et dont la température initiale de cuve est d'environ 90°C, le ou les élastomères diéniques, la ou les charges renforçantes, l'agent de couplage éventuel puis, après une à deux minutes de malaxage, les divers autres ingrédients à l'exception du système de vulcanisation. On conduit alors un travail thermomécanique (phase non-productive) en une étape (durée totale du malaxage égale à environ 5 min), jusqu'à atteindre une température maximale de "tombée" d'environ 165°C. On récupère le mélange ainsi obtenu, on le refroidit puis on ajoute le système de vulcanisation (soufre et composé triazine (ou « CBS » pour l'exemple comparatif)) sur un mélangeur externe (homo-finisseur) à 70°C, en mélangeant le tout (phase productive) pendant environ 5 à 6 min.

Les compositions ainsi obtenues sont ensuite calandrées soit sous la forme de plaques (épaisseur de 2 à 3 mm) ou de feuilles fines de caoutchouc pour la mesure de leurs propriétés physiques ou mécaniques, soit sous la forme de profilés utilisables directement, après découpage et/ou assemblage aux dimensions souhaitées, par exemple comme produits semi-finis pour pneumatiques, en particulier comme bandes de roulement de pneumatiques.

### III-1.3. Essais de caractérisation - Résultats

### A- Exemple 1

L'objet de cet exemple est de comparer les propriétés d'une composition de caoutchouc comprenant du noir de carbone à titre de charge renforçante majoritaire, utilisable pour la fabrication d'une bande de roulement de pneumatique, comprenant la 3-[(cyclohexylamino)thio]-5-phényl-1,2,4-triazine (composé B) à titre d'accélérateur primaire de vulcanisation (composition 2) avec les propriétés d'une composition de caoutchouc comprenant la N-cyclohexyl-2-benzothiazyle sulfénamide (« CBS ») (composition 1).

Les formulations des compositions sont données dans le tableau 2. Les quantités sont exprimées en parties pour 100 parties en poids d'élastomère (pce).

**Tableau 2**

| | Composition 1 | Composition 2 |
|---|---|---|
| NR (1) | 40 | 40 |
| BR (2) | 20 | 20 |
| SBR (3) | 40 | 40 |
| N234 (4) | 54 | 54 |
| Paraffine | 1 | 1 |
| 6-PPD (5) | 2 | 2 |
| Acide stéarique | 2 | 2 |
| ZnO | 2,7 | 2,7 |
| Soufre | 1,1 | 1,1 |
| Accélérateur de vulcanisation | 1,1 * | 1,23 ** |

| | | |
|---|---|---|
| * CBS (« Santocure CBS » de la société Flexsys) ** 3-[(cyclohexylamino)thio]-5-phényl-1,2,4-triazine (1) caoutchouc naturel (2) polybutadiène avec 0,7% de 1-2 ; 1,7% de trans 1-4 ; 98% de cis 1-4 (Tg = -105°C) (% molaires) (3) copolymère butadiène-styrène SSBR (SBR préparé en solution) avec 25% de styrène, 59% de motifs polybutadiène 1-2 et 20% de motifs polybutadiène 1-4 trans (Tg = -24°C) (% molaires) ; taux exprimé en SBR sec (SBR étendu avec 9% en poids d'huile MES, soit un total de SSBR + huile égal à 76 pce) (4) noir de carbone N 234 (5) Agent anti-oxydant 6-para-phénylènediamine | | |

La composition de caoutchouc 2 comprenant la 3-[(cyclohexylamino)thio]-5-phényl-1,2,4-triazine est identique à la composition 1, étant entendu que la CBS est remplacée par une quantité isomolaire de 3-[(cyclohexylamino)thio]-5-phényl-1,2,4-triazine.

Les propriétés rhéométriques à 150°C sont données dans le tableau 3.

**Tableau 3**

| | Composition 1 (CBS) | Composition 2 (Composé B) |
|---|---|---|
| Prop.rhéo. *(DIN)* | 150°C | |
| Δcouple(dN.m) | 8,5 | 8,7 |
| k(min⁻¹) | 0,416 | 0,353 |
| t₀(min) | 6,0 | 8,6 |

Les propriétés rhéométriques obtenues pour la composition 2 contenant la 3-[(cyclohexylamino)thio]-5-phényl-1,2,4-triazine sont équivalentes à celles obtenues pour la composition 1 contenant la CBS. On note même que la phase retard (délai d'induction t₀) est plus long dans le cas de la composition 2 contenant la 3-[(cyclohexylamino)thio]-5-phényl-1,2,4-triazine, ce qui est avantageux.

On note, par ailleurs que le composé B, ainsi que les composés de formule (I) en général, remplacent avantageusement vis-à-vis de l'impact environnemental, les sulfénamides à noyau mercaptobenzothiazole, en ne générant pas contrairement à ces derniers, de mercaptobenzothiazole en se décomposant au cours de la cuisson.

### B-Exemple 2

L'objet de cet exemple est de comparer les propriétés d'une composition de caoutchouc comprenant de la silice à titre de charge renforçante majoritaire, utilisable pour la fabrication d'une bande de roulement de pneumatique, comprenant la 3-[(cyclohexylamino)thio]-5-phényl-1,2,4-triazine (composé B) à titre d'accélérateur primaire de vulcanisation (composition 4) avec les propriétés d'une composition de caoutchouc comprenant la N-cyclohexyl-2-benzothiazyle sulfénamide (« CBS ») (composition 3).

Les formulations des compositions sont données dans le tableau 4. Les quantités sont exprimées en parties pour 100 parties en poids d'élastomère (pce).

**Tableau 4**

| | Composition 3 | Composition 4 |
|---|---|---|
| BR (1) | 28 | 28 |
| SBR (2) | 79,2 | 79,2 |
| N234 (3) | 4 | 4 |
| Silice (4) | 82 | 82 |
| 6-PPD (5) | 1,9 | 1,9 |
| MES (6) | 4,8 | 4,8 |
| Cire anti-ozone | 1,5 | 1,5 |
| Résine plastifiante (7) | 20 | 20 |
| Agent de couplage (8) | 6,56 | 6,56 |
| Acide stéarique | 2 | 2 |
| DPG (9) | 1,54 | 1,54 |
| ZnO | 1,5 | 1,5 |
| Soufre | 1,2 | 1,2 |
| Accélérateur de vulcanisation | 1,9* | 2,12** |

| | | |
|---|---|---|
| * CBS (« Santocure CBS » de la société Flexsys) ** 3-[(cyclohexylamino)thio]-5-phényl-1,2,4-triazine (1) polybutadiène avec 0,7% de 1-2 ; 1,7% de trans 1-4 ; 98% de cis 1-4 (Tg = -105°C) (% molaires) (2)copolymère butadiène-styrène SSBR (SBR préparé en solution) avec 25% de styrène, 59% de motifs polybutadiène 1-2 et 20% de motifs polybutadiène 1-4 trans (Tg = -24°C) (% molaires); taux exprimé en SBR sec (SBR étendu avec 9% en poids d'huile MES, soit un total de SSBR + huile égal à 76 pce) (3) noir de carbone N234 (4) Silice "Zeosil 1165MP" de la société Rhodia, type "HDS" (BET et CTAB : environ 160 m²/g); (5)Agent anti-oxydant 6-para-phénylènediamine (6) Huile plastifiante « Medium Extracted Solvates » (Catenex SNR de Shell) (7) Résine aliphatique (pure C5) « Hikorez A-1100 » commercialisée par la société KOLON (8)Agent de couplage tétrasulfure de bis(3-triéthoxysilylpropyl, TESPT ("Si69" de la société Degussa (9)Diphénylguanidine (Perkacit DPG de la société Flexsys) | | |

La composition de caoutchouc 4 comprenant la 3-[(cyclohexylamino)thio]-5-phényl-1,2,4-triazine est identique à la composition 3, étant entendu que la CBS est remplacée par une quantité isomolaire de 3-[(cyclohexylamino)thio]-5-phényl-1,2,4-triazine.

Les propriétés rhéométriques à 150°C sont données dans le tableau 5.

**Tableau 5**

| | Composition 3 (CBS) | Composition 4 (Composé B) |
|---|---|---|
| Prop.rhéo. *(DIN)* | 150°C | |
| Δcouple(dN.m) | 11,9 | 12,2 |
| k(min⁻¹) | 0,064 | 0,052 |
| t₀(min) | 4,9 | 6,9 |

Les propriétés rhéométriques obtenues pour la composition 4 contenant la 3-[(cyclohexylamino)thio]-5-phényl-1,2,4-triazine sont équivalentes à celles obtenues pour la composition 1 contenant la CBS. On note même que la phase retard (délai d'induction t₀) est plus long dans le cas de la composition 4 contenant la 3-[(cyclohexylamino)thio]-5-phényl-1,2,4-triazine, ce qui est avantageux.

### III-2. La 3-[(t-butylamino)thio]-5-phényl-1,2,4-triazine sulfénamide

On décrit ici le procédé de préparation de la 3-[(t-butylamino)thio]-5-phényl-1,2,4-triazine sulfénamide (composé C) de formule suivante :

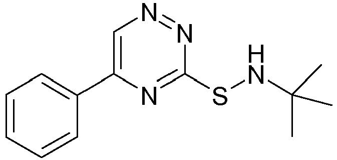

La préparation de cette sulfénamide repose sur un couplage oxydant entre le 5-phényl-1,2,4-triazine-3-thiol et la tertbutylamine, selon le schéma de synthèse suivant :

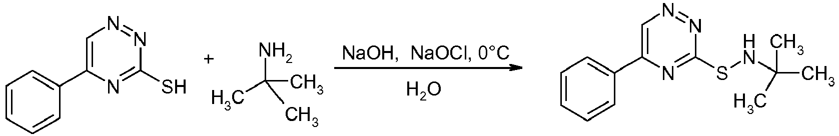

A 350 mL de tert-butylamine maintenue entre -10°C et -5 °C (température du bain), est ajoutée goutte à goutte une solution aqueuse de NaOCl (136 mL, titrée à 17,4 % de chlore actif) pendant 45 min. Pendant toute la durée d'addition la température du bain est maintenue entre -10°C et -5°C. A cette solution maintenue à 0°C, est ajouté goutte à goutte pendant 90 min une solution de 5-phényl-1,2,4-triazine-3-thiol (40,30 g, 0,213 mol; pureté : environ 98 % molaire. déterminée par RMN), d'hydroxyde de sodium (16,96 g, 0,424 mol) et de tert-butylamine (50 ml) dans l'eau (350 mL). La température du milieu réactionnel reste maintenue entre 0 et +6°C. Le milieu réactionnel est ensuite agité pendant une heure à température entre +5 et +10°C, puis 2 heures à température ambiante.

Ensuite on dilue ce milieu par 0,7 L d'eau froide (environ 4 °C). Cette suspension est encore agitée pendant 10 minutes puis le précipité du produit obtenu est filtré et lavé sur le filtre par de l'eau (10 fois 500 mL) puis séché 48 h sous air. Un solide jaune (49,5 g, 0,190 mol, rendement 89 %) de point de fusion 73 °C est obtenu. La pureté molaire déterminée par RMN ¹H est de 95%.

Le suivi de la réaction est réalisé par chromatographie sur couche mince :
Rf_{produit} = 0,52, Rf_{impureté disulfure} = 0,61 (caractéristiques de la CCM : SiO₂; EtOAc : heptane = 1 : 1; révélation par UV et I₂).

La caractérisation complète du produit est réalisée par RMN. Les déplacements chimiques obtenus par RMN ¹H et ¹³C dans le DMSO-d6 sont donnés dans le tableau ci-dessous. La calibration est réalisée sur le DMSO (2,44 ppm en ¹H et à 39,5 ppm en ¹³C).

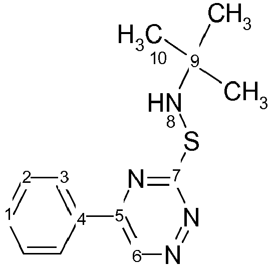

Les résultats sont donnés dans le tableau 6.

**Tableau 6**

| N° | 1 | 2 | 3 | 4 | 5 | 6 | 7 | 8 | 9 | 10 |
|---|---|---|---|---|---|---|---|---|---|---|
| δ¹H ppm | 7,59 | 7,56 | 8,29 | - | - | 9,72 | - | 4,87 | - | 1,11 |
| δ¹³C ppm | 132,7 | 128,9 | 127,7 | 129,7 | 153,7 | 142,9 | 177,0 | - | 54,3 | 29,1 |

## Revendications

1. 1,2,4-Triazine de formule (I) : 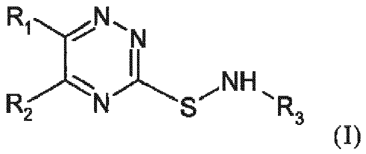 où
R₁ et R₂ représentent indépendamment H ou un groupe hydrocarboné en C₁-C₂₅ choisi parmi les groupes alkyles linéaires, ramifiés ou cycliques, et les groupes aryles, R₁ et R₂ pouvant former ensemble un cycle,
R₃ représente :
- un groupe alkyle en C₁-C₂₅, linéaire ou ramifié, éventuellement substitué par un ou plusieurs groupes alkyles cycliques en C₃-C₁₀ ou aryles en C₆-C₁₂, ou
- un groupe alkyle cyclique en C₃-C₁₀, éventuellement substitué par un ou plusieurs groupes alkyles linéaires, ramifiés ou cycliques en C₁-C₂₅ ou aryles en C₆-C₁₂.

2. 1,2,4-Triazine selon la revendication 1 **caractérisée en ce que** R₁ et R₂ représentent indépendamment H, un groupe méthyle ou un groupe phényle.

3. 1,2,4-Triazine selon la revendication 2 **caractérisée en ce que** R₁ ou R₂ représentent un groupe phényle.

4. 1,2,4-Triazine selon la revendication 3 **caractérisée en ce que** R₁ représente un hydrogène et R₂ est un groupe phényle.

5. 1,2,4-Triazine selon l'une quelconque des revendications précédentes **caractérisée en ce que** R₃ représente un groupe cyclohexyle ou un groupe tertbutyle.

6. 1,2,4-Triazine selon l'une quelconque des revendications précédentes **caractérisée en ce que** R₃ représente un groupe tertbutyle.

7. Utilisation, à titre d'accélérateur de vulcanisation, d'une 1,2,4-triazine telle que définie à l'une quelconque des revendications précédentes.

8. Procédé de préparation d'une 1,2,4-triazine telle que définie dans l'une quelconque des revendications 1 à 6, par couplage oxydant entre
- un composé 1,2,4-triazine-3-thiol de formule (A) suivante : 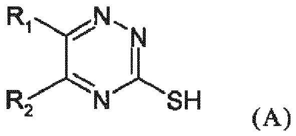 où R₁ et R₂ sont tels que définis dans l'une quelconque des revendications 1 à 4, et
- une amine primaire de formule R₃NH₂, où R₃ est tel que défini dans la revendication 1, 5 ou 6,
le couplage oxydant étant réalisé au moyen d'une composition basique et d'un composé oxydant.

9. Procédé selon la revendication 8, **caractérisé en ce qu'**il comprend les étapes suivantes :
- on fait réagir le composé (A) avec ladite composition basique, puis
- on ajoute au milieu réactionnel ladite amine primaire de formule R₃NH₂, puis
- on réalise le couplage oxydant par ajout dudit composé oxydant.

10. Procédé selon la revendication 8, **caractérisé en ce qu'**il comprend les étapes suivantes :
a) on fait réagir de l'amine primaire de formule R₃NH₂ avec ledit composé oxydant, puis
b) on ajoute au milieu réactionnel obtenu à l'étape a) une composition comprenant la composition basique, ledit composé de formule (A) et de l'amine primaire de formule R₃NH₂**.**

11. Procédé selon la revendication 10 **caractérisé en ce que** R₃ est un groupe tertbutyle.

12. Procédé selon l'une quelconque des revendications 8 à 11 **caractérisé en ce que** la composition basique est une solution aqueuse d'une base organique ou minérale.

13. Procédé selon la revendication 12 **caractérisé en ce que** la base minérale est l'hydroxyde de sodium.

14. Procédé selon l'une quelconque des revendications 8 à 13 **caractérisé en ce que** le composé oxydant est choisi parmi les composés halogénés, de préférence le dichlore, le dibrome, le diiode et les acides hypohalogéneux et leurs sels alcalins.

15. Procédé selon la revendication 14 **caractérisé en ce que** le composé oxydant est l'hypochlorite de sodium.

## Patentansprüche

1. 1,2,4-Triazin der Formel (I): 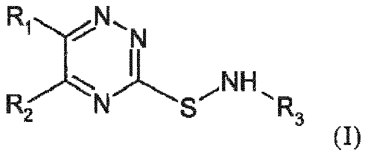 wobei
R₁ und R₂ jeweils unabhängig für H oder eine C₁-C₂₅-Kohlenwasserstoffgruppe, die aus linearen, verzweigten oder cyclischen Alkylgruppen und Arylgruppen ausgewählt ist, stehen, wobei R₁ und R₂ zusammen einen Ring bilden können,
R₃ für:
- eine lineare oder verzweigte C₁-C₂₅-Alkylgruppe, die gegebenenfalls durch eine oder mehrere cyclische C₃-C₁₀-Alkyl- oder C₆-C₁₂-Arylgruppen substituiert ist, oder
- eine cyclische C₃-C₁₀-Alkylgruppe, die gegebenenfalls durch eine oder mehrere lineare, verzweigte oder cyclische C₁-C₂₅-Alkylgruppen oder C₆-C₁₂-Arylgruppen substituiert ist,
steht.

2. 1,2,4-Triazin nach Anspruch 1, **dadurch gekennzeichnet, dass** R₁ und R₂ unabhängig für H, eine Methylgruppe oder eine Phenylgruppe stehen.

3. 1,2,4-Triazin nach Anspruch 2, **dadurch gekennzeichnet, dass** R₁ oder R₂ für eine Phenylgruppe steht.

4. 1,2,4-Triazin nach Anspruch 3, **dadurch gekennzeichnet, dass** R₁ für Wasserstoff steht und R₂ für eine Phenylgruppe steht.

5. 1,2,4-Triazin nach einem der vorhergehenden Ansprüche, **dadurch gekennzeichnet, dass** R₃ für eine Cyclohexylgruppe oder eine tert-Butylgruppe steht.

6. 1,2,4-Triazin nach einem der vorhergehenden Ansprüche, **dadurch gekennzeichnet, dass** R₃ für eine tert-Butylgruppe steht.

7. Verwendung eines 1,2,4-Triazins gemäß einem der vorhergehenden Ansprüche als Vulkanisations-beschleuniger.

8. Verfahren zur Herstellung eines 1,2,4-Triazins gemäß einem der Ansprüche 1 bis 6 durch oxidative Kupplung zwischen
- einer 1,2,4-Triazin-3-thiol-Verbindung der folgenden Formel (A): 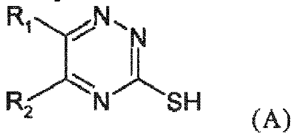 wobei R₁ und R₂ wie in einem der Ansprüche 1 bis 4 definiert sind, und
- einem primären Amin der Formel R₃NH₂, wobei R₃ wie in Anspruch 1, 5 oder 6 definiert ist,
wobei die oxidative Kupplung mit Hilfe einer basischen Zusammensetzung und einer oxidierenden Verbindung durchgeführt wird.

9. Verfahren nach Anspruch 8, **dadurch gekennzeichnet, dass** es folgende Schritte umfasst:
- Umsetzen der Verbindung (A) mit der basischen Zusammensetzung, dann
- Zugeben des primären Amins der Formel R₃NH₂ zum Reaktionsmedium, dann
- Durchführen der oxidativen Kupplung durch Zugabe der oxidierenden Verbindung.

10. Verfahren nach Anspruch 8, **dadurch gekennzeichnet, dass** es folgende Schritte umfasst:
a) Umsetzen des primären Amins der Formel R₃NH₂ mit der oxidierenden Verbindung, dann
b) Zugeben einer Zusammensetzung, die die basische Zusammensetzung, die Verbindung der Formel (A) und das primäre Amin der Formel R₃NH₂ umfasst, zu dem in Schritt a) erhaltenen Reaktionsmedium.

11. Verfahren nach Anspruch 10, **dadurch gekennzeichnet, dass** es sich bei R₃ um eine tert-Butylgruppe handelt.

12. Verfahren nach einem der Ansprüche 8 bis 11, **dadurch gekennzeichnet, dass** es sich bei der basischen Zusammensetzung um eine wässrige Lösung einer organischen oder anorganischen Base handelt.

13. Verfahren nach Anspruch 12, **dadurch gekennzeichnet, dass** es sich bei der anorganischen Base um Natriumhydroxid handelt.

14. Verfahren nach einem der Ansprüche 8 bis 13, **dadurch gekennzeichnet, dass** die oxidierende Verbindung aus Halogenverbindungen, vorzugsweise Dichlor, Dibrom und Diiod und hypohalogenigen Säuren und Alkalisalzen davon, ausgewählt wird.

15. Verfahren nach Anspruch 14, **dadurch gekennzeichnet, dass** es sich bei der oxidierenden Verbindung um Natriumhypochlorit handelt.

## Claims

1. 1,2,4-Triazine of formula (I): 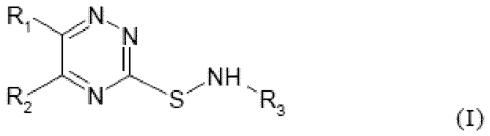 where
R₁ and R₂ independently represent H or a C₁-C₂₅ hydrocarbon group chosen from linear, branched or cyclic alkyl groups, and aryl groups, it being possible for R₁ and R₂ to together form a ring,
R₃ represents:
- a linear or branched C₁-C₂₅ alkyl group which is optionally substituted by one or more cyclic C₃-C₁₀ alkyl or C₆-C₁₂ aryl groups, or
- a cyclic C₃-C₁₀ alkyl group which is optionally substituted by one or more linear, branched or cyclic C₁-C₂₅ alkyl or C₆-C₁₂ aryl groups.

2. 1,2,4-Triazine according to Claim 1, **characterized in that** R₁ and R₂ independently represent H, a methyl group or a phenyl group.

3. 1,2,4-Triazine according to Claim 2, **characterized in that** R₁ or R₂ represent a phenyl group.

4. 1,2,4-Triazine according to Claim 3, **characterized in that** R₁ represents a hydrogen and R₂ is a phenyl group.

5. 1,2,4-Triazine according to any one of the preceding claims, **characterized in that** R₃ represents a cyclohexyl group or a tert-butyl group.

6. 1,2,4-Triazine according to any one of the preceding claims, **characterized in that** R₃ represents a tert-butyl group.

7. Use, as vulcanization accelerator, of a 1,2,4-triazine as defined in any one of the preceding claims.

8. Process for the preparation of a 1,2,4-triazine as defined in any one of Claims 1 to 6, by oxidative coupling between
- a 1,2,4-triazine-3-thiol compound of following formula (A): 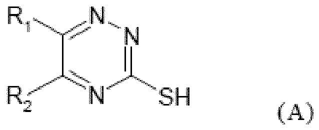 where R₁ and R₂ are as defined in any one of Claims 1 to 4, and
- a primary amine of formula R₃NH₂, where R₃ is as defined in Claim 1, 5 or 6,
the oxidative coupling being carried out using a basic composition and an oxidizing compound.

9. Process according to Claim 8, **characterized in that** it comprises the following stages:
- the compound (A) is reacted with the said basic composition, then
- the said primary amine of formula R₃NH₂ is added to the reaction medium, then
- the oxidative coupling is carried out by addition of the said oxidizing compound.

10. Process according to Claim 8, **characterized in that** it comprises the following stages:
a) primary amine of formula R₃NH₂ is reacted with the said oxidizing compound, then
b) a composition comprising the basic composition, the said compound of formula (A) and primary amine of formula R₃NH₂ is added to the reaction medium obtained in stage a).

11. Process according to Claim 10, **characterized in that** R₃ is a tert-butyl group.

12. Process according to any one of Claims 8 to 11, **characterized in that** the basic composition is an aqueous solution of an organic or inorganic base.

13. Process according to Claim 12, **characterized in that** the inorganic base is sodium hydroxide.

14. Process according to any one of Claims 8 to 13, **characterized in that** the oxidizing compound is chosen from halogen compounds, preferably chlorine, bromine, iodine and hypohalous acids and their alkali metal salts.

15. Process according to Claim 14, **characterized in that** the oxidizing compound is sodium hypochlorite.
